# EUROPEAN PATENT SPECIFICATION

(11) **EP 2 106 739 B1**
(45) Date of publication and mention of the grant of the patent: **11.05.2016**
(21) Application number: 09005059.2
(22) Date of filing: 06.04.2009
(51) Int. Cl.: A61B 1/12, G02B 23/24, G02B 27/00

(54) **Endoscope, distal end cap-equipped endoscope and endoscope cleaning sheath**
Endoskop, am distalen Ende mit einer Kappe ausgerüstetes Endoskop und Endoskopreinigungshülle
Endoscope, endoscope équipé d'un couvercle à extrémité distale et gaine de nettoyage d'endoscope

(30) Priority: 04.04.2008 JP 2008098558
(43) Date of publication of application: 07.10.2009
(73) Proprietor: Olympus Corporation, Shibuya-ku Tokyo (JP)
(72) Inventor: Miyamoto, Shinichi, Hachioji-shi Tokyo 192-8512 (JP)
(74) Representative: von Hellfeld, Axel

(56) References cited:
- WO-A-01/89371
- DE-A1-102004 029 099
- JP-A- 6 014 870
- US-A- 5 688 221
- US-A1- 2008 188 715

## Description

The present invention relates to an endoscope including a fluid jet nozzle for cleaning, e.g. contamination adhering to an observation window which is provided on a distal end section body of the endoscope, a distal end cap-equipped endoscope, and an endoscope cleaning sheath.

In a medical endoscope, an insertion section, which is inserted into a body cavity, is provided with an illumination window and an observation window at a distal end section body thereof. While emitting illumination light from the illumination window and illuminating the body cavity, observation is performed through the observation window. The distal end section body is provided with a fluid jet nozzle. In a case where blood, mucus or the like adheres to the illumination window or observation window and the field of vision is deteriorated, water and air are jetted from the fluid jet nozzle, thereby to clean the illumination window or observation window.

Jpn. Pat. Appln. KOKAI Publication No. H11-188004 (patent document 1) discloses the following structure. A distal end cap is detachably attached to a distal end section body of an insertion section of an endoscope. This distal end cap includes the air/water feed nozzle as mentioned above.

An air feed path and a water feed path are formed in the distal end section body. Distal end portions of a water feed tube and an air feed tube are connected, respectively, to the air feed path and the water feed path. A communication path, at which the air feed path and the water feed path are made confluent, is provided in the distal end section body. The air/water feed nozzle is connected to the communication path.

Proximal end portions of the water feed tube and air feed tube are connected to water feed means and air feed means on the proximal side of the insertion section. Water and air are supplied to the water feed tube and air feed tube from the water feed means and air feed means on the proximal side of the insertion section. In this structure, the water, which is fed from the water feed tube, and the air, which is fed from the air feed tube, are supplied to the air/water feed nozzle via the communication path in the distal end section body, and the water and air are jetted from the air/water feed nozzle to the observation window.

Jpn. Pat. Appln. KOKAI Publication No. H10-151108 (patent document 2), like patent document 1, discloses a structure wherein a water feed tube and an air feed tube are connected to a water feed path and an air feed path in a distal end section body of an endoscope, and the water feed tube and the air feed tube are made confluent in a communication path which is provided in the distal end section body. In addition, an air/water feed nozzle having a distal end portion with a reduced diameter is connected to the communication path. In this structure, water and air are jetted from the air/water feed nozzle to the observation window.

Jpn. Pat. Appln. KOKAI Publication No. H7-136102 (patent document 3) discloses the following structure. An air feed outlet and a water feed outlet, which open at a distal end face of a distal end potion body of an insertion section of an endoscope, are provided adjacent to each other. In the distal end section body, a nozzle is detachably attached in such a manner that the nozzle is opposed to the air feed outlet and water feed outlet. In this structure, the direction of jet of the air, which is fed from the air feed outlet, and the direction of jet of the water, which is fed from the water feed outlet, are varied by the nozzle, and the air and water are jetted toward the observation window.

Jpn. Pat. Appln. KOKAI Publication No. H6-14870 (patent document 4) discloses the following structure. An air feed path and a water feed path are provided in an insertion section of an endoscope. The air feed path and water feed path are made confluent in the insertion section, and made to communicate with an air/water feed nozzle. Further, air is intermittently blown into the water flowing in the water feed path, thus producing an air/water mixture fluid and enhancing the performance of cleaning.

In the structures of the above-described patent documents 1 and 2, the water feed tube and air feed tube are made confluent in the communication path that is provided in the distal end section body. However, the water feed and air feed are not performed at the same time. This structure is provided with a function of feeding water and a function of feeding air toward the observation window from the air/water feed nozzle. In short, in patent documents 1 and 2, there is no technical idea that water and air are mixed into an atomized gas/liquid mixture in the communication path, and this atomized gas/liquid mixture fluid is jetted toward the observation window.

In patent document 3, the air feed outlet and water feed outlet are provided adjacent to each other at the distal end section body. In this structure, the direction of the air that is fed from the air feed outlet and the direction of the water that is fed from the water feed outlet are deflected by the nozzle, and the water and air are jetted to the observation window. In this case, the air and water are selectively jetted to the observation window.

Further, in patent document 3, the air feed outlet is provided at a position near the observation window, and the water feed outlet is provided at a position far from the observation window. Thus, even if the air and water are simultaneously fed from the air feed outlet and water feed outlet, it is not possible to blow the water into atomized water by the jet pressure. In patent document 3, there is no technical idea that water and air are mixed into an atomized gas/liquid mixture, and this atomized gas/liquid mixture fluid is jetted toward the observation window.

As has been described above, in each of patent documents 1 to 3, one of air and water is selectively jetted toward the observation window. None of patent documents 1 to 3 discloses the technical idea that when contamination adheres to the observation window and the field of vision is deteriorated, water and air are mixed into an atomized air/water mixture, and the atomized air/water mixture is jetted to the observation window, thereby cleaning the observation window. Thus, in some cases, in order to secure the cleaning effect, the time in which water and air are jetted needs to be increased. In such cases, there arises a problem that the consumption of water and air increases accordingly.

Patent document 4 describes that the cleaning power is enhanced by producing an air/water mixture fluid. Patent document 4 discloses the structure in which air is intermittently blown into water by opening/closing the air feed valve, thus producing an air/water mixture.

In addition, in paten documents 1 to 4, air and water are used, or air and water are mixed into an air/water mixture fluid, thereby to jet the air/water to the observation window from the jet port. However, none of patent documents 1 to 4 discloses the technique for making the air/water mixture fluid reach the entirety of the observation window. Moreover, it is clear from experiments that when air and water are to be mixed, the water and air are easily mixed if the supply amount of air is made greater than the supply amount of water. However, in the case where the supply amount of air is made greater than the supply amount of water and the air/water mixture fluid is jetted from the jet port, the air/water mixture fluid is not jetted straight from the jet port, and such a problem arises that the air/water mixture fluid tends to be biased to the water with the less supply amount, and the air/water mixture fluid does not reach the entirety of the observation window. Thus, even if it is assumed that in patent document 4, too, the water and air are mixed into an atomized air/water mixture that is optimal for cleaning, and the atomized air/water mixture is jetted to the observation window, it would be necessary to make some device for obtaining the cleaning effect as described above.

Besides, in the case where a surgical operation is performed by a rigid endoscope while feeding air into a body cavity in pneumoperitoneum during the surgical operation, the amount of air that is fed into the body cavity increases. If excessive air feed is performed, the load on the patient increases. It is thus desirable that the amount of air that is fed into the body cavity is as small as possible. In such a case, it is necessary to manage the time of the surgical operation so that the time for cleaning may not increase, that is, the amount of air that is fed into the body cavity may not increase.

The present invention has been made in consideration of the above-described circumstances, and the object of the invention is to provide an endoscope, a distal end cap-equipped endoscope and an endoscope cleaning sheath, which can mix a liquid from a liquid feed path and a gas from a gas feed path into an atomized liquid/gas mixture fluid, and can jet the atomized liquid/gas mixture fluid to an observation window, thereby efficiently cleaning the observation window.

According to a first aspect of the present invention, an endoscope comprising: an insertion section which is inserted in a body cavity; a distal end section body which constitutes a distal end section of the insertion section and has at least an observation window; a liquid feed path which is formed to supply a liquid to the distal end section body side and communicates with a liquid feed source; a gas feed path which is formed to supply a gas to the distal end section body side and communicates with a gas feed source; and a nozzle which is provided in the distal end section body and cleans the observation window by jetting toward the observation window a mixture fluid in which the liquid supplied from the liquid feed path and the gas supplied from the gas feed path are mixed, wherein the nozzle includes: a confluent portion which is provided in a mount plane in which the observation window of the distal end section body is provided, or in a plane parallel to the mount plane, the confluent portion making confluent and mixing the liquid supplied from the liquid feed path and the gas supplied from the gas feed path; and a jet outlet which jets a gas/liquid mixture fluid, which is mixed in the confluent portion, toward the observation window.

According to another aspect of the present invention, a distal end cap-equipped endoscope comprising: an insertion section which is inserted in a body cavity; a distal end section body which constitutes a distal end section of the insertion section and has at least an observation window; a liquid feed path which is formed to supply a liquid to the distal end section body side and communicates with a liquid feed source; a gas feed path which is formed to supply a gas to the distal end section body side and communicates with a gas feed source; a distal end cap which is detachably attached to the distal end section body; and a nozzle which is provided in the distal end cap and cleans the observation window by jetting toward the observation window a mixture fluid in which the liquid supplied from the liquid feed path and the gas supplied from the gas feed path are mixed, wherein the nozzle includes a confluent portion which makes confluent and mixes the liquid supplied from the liquid feed path and the gas supplied from the gas feed path, and a jet outlet which jets a gas/liquid mixture fluid, which is mixed in the confluent portion, toward the observation window.

According to another aspect of the present invention, an endoscope cleaning sheath comprising: a cleaning sheath body which is fitted over an insertion section of an endoscope having at least an observation window at a distal end section body; a liquid feed path which is provided in the cleaning sheath body and communicates with a liquid feed source; a gas feed path which is provided in the cleaning sheath body and communicates with a gas feed source; and a nozzle which is provided in the cleaning sheath body and cleans the observation window by jetting toward the observation window a mixture fluid in which the liquid supplied from the liquid feed path and the gas supplied from the gas feed path are mixed, wherein the nozzle includes: a confluent portion which makes confluent and mixes the liquid supplied from the liquid feed path and the gas supplied from the gas feed path; and a jet outlet which jets a gas/liquid mixture fluid, which is mixed in the confluent portion, toward the observation window.

The present invention has the advantageous effect that a liquid from a liquid feed path and a gas from a gas feed path can efficiently be mixed into an atomized liquid/gas mixture fluid, and the atomized liquid/gas mixture fluid can be jetted to an observation window, thereby efficiently cleaning the observation window.

The invention can be more fully understood from the following detailed description when taken in conjunction with the accompanying drawings, in which:
FIG. 1A is a perspective view showing the entire structure of an endoscope and an endoscope cleaning sheath according to a first embodiment not part of the present invention;
FIG. 1B is a front view of a distal end section body of the endoscope;
FIG. 2 is a transverse cross-sectional view of a sheath body of the endoscope of the first embodiment;
FIG. 3 is a longitudinal cross-sectional side view of the distal end section of the endoscope of the first embodiment;
FIG. 4 is a front view showing the distal end section of the endoscope of the first embodiment;
FIG. 5A is a cross-sectional view taken along line 5A-5A in FIG. 3;
FIG. 5B is a cross-sectional view showing a modification of the structure of FIG. 5A;
FIG. 6 is a perspective view of a flexible endoscope according to a second embodiment not part of the present invention;
FIG. 7 is a perspective view of a distal end portion of an endoscope according to the second embodiment;
FIG. 8 is a longitudinal cross-sectional side view of the distal end portion of the endoscope according to the second embodiment;
FIG. 9A is a schematic front view showing a first modification of the nozzle of the second embodiment;
FIG. 9B is a schematic front view showing a second modification of the nozzle of the second embodiment;
FIG. 9C is a schematic front view showing a third modification of the nozzle of the second embodiment;
FIG. 9D is a schematic front view showing a fourth modification of the nozzle of the second embodiment;
FIG. 9E is a schematic front view showing a fifth modification of the nozzle of the second embodiment;
FIG. 9F is a schematic front view showing a sixth modification of the nozzle of the second embodiment;
FIG. 9G is a schematic front view showing a seventh modification of the nozzle of the second embodiment;
FIG. 9H is a schematic front view showing an eighth modification of the nozzle of the second embodiment;
FIG. 10 is a perspective view of a nozzle of a distal end portion according to a third embodiment not part of the present invention;
FIG. 11 is a front view of the nozzle of the distal end section body in the third embodiment;
FIG. 12A is a front view of a distal end section body of an endoscope according to a fourth embodiment not part of the present invention;
FIG. 12B is a cross-sectional view taken along line 12B-12B in FIG. 12A;
FIG. 13A is a front view of a distal end section body of an endoscope according to a fifth embodiment not part of the present invention;
FIG. 13B is a cross-sectional view taken along line 13B-13B in FIG. 13A;
FIG. 14A is a front view of a distal end section body of an endoscope according to a sixth embodiment not part of the present invention;
FIG. 14B is a longitudinal cross-sectional side view of the distal end section body;
FIG. 15A is a front view of a distal end section body of an endoscope according to a seventh embodiment not part of the present invention;
FIG. 15B is a longitudinal cross-sectional side view of the distal end section body;
FIG. 16A is a front view of a distal end section body according to an eighth embodiment of the present invention;
FIG. 16B is a longitudinal cross-sectional side view of the distal end section body;
FIG. 17A is a front view of a distal end section body according to a ninth embodiment of the present invention;
FIG. 17B is a longitudinal cross-sectional side view of the distal end section body;
FIG. 18 is a perspective view of a distal end cap-equipped endoscope according to a tenth embodiment not part of the invention;
FIG. 19 is a longitudinal cross-sectional side view of a distal end section body of the distal end cap-equipped endoscope according to the tenth embodiment;
FIG. 20 is a cross-sectional view taken along line 20-20 in FIG. 19; and
FIG. 21 is a cross-sectional view taken along line 21-21 in FIG. 19.

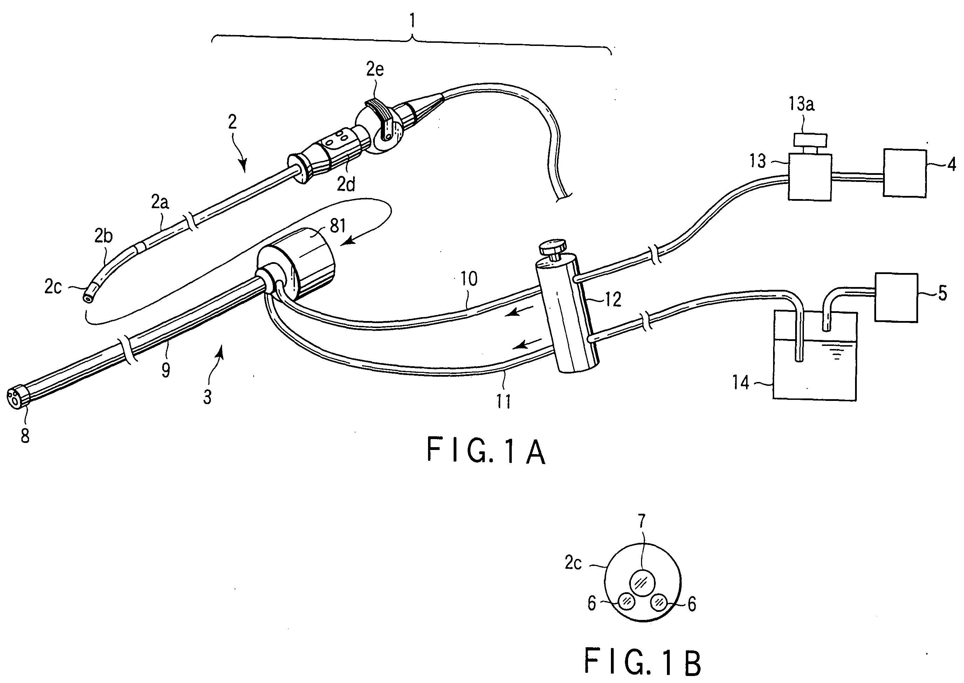

Embodiments of the present invention will now be described with reference to the accompanying drawings. FIG. 1A to FIG. 5A show a first embodiment. FIG. 1A is a perspective view showing the entire structure of an endoscope apparatus 1, and FIG. 1B is a front view of a distal end section body 2c of an endoscope 2.

As shown in FIG. 1A, the endoscope apparatus 1 comprises the endoscope 2, an endoscope cleaning sheath 3, a gas feed pump 4 functioning as a gas feed device, and a liquid feed pump 5 functioning as a liquid feed device. The endoscope 2 is, for example, a rigid endoscope having a bending section 2b in an insertion section 2a thereof. A distal end portion of the insertion section 2a is provided with a distal end section body 2c. A proximal end portion of the insertion section 2a is provided with an operation section 2d. The operation section 2d is provided with a bending operation lever 2e for bending the bending section 2b in an up-and-down direction or in a right-and-left direction.

The endoscope cleaning sheath 3 is fitted over the insertion section 2a of the endoscope 2. Thereby, the endoscope cleaning sheath 3, as one piece with the insertion section 2a, is inserted into a body cavity. As shown in FIG. 1B, a distal end face of the distal end section body 2c is provided with two illumination windows 6 and one observation window 7. The illumination widows 6 constitute parts of an illumination optical system. The observation window 7 constitutes a part of an observation optical system.

The illumination window 6 is connected to a light source device (not shown) via a light guide fiber. The observation optical system is provided with an image pickup device including an image pickup element, such as a CCD, which photoelectrically converts an optical image, which is captured through the observation window 7, to an electric signal. A signal cable extends from the image pickup device. This signal cable is connected to an external camera control unit (not shown). Thus, reflective light from a subject, which is illuminated with illumination light that is emitted from the illumination window 6, is received as an optical image via the observation window 7. The optical image, after converted to the electric signal by the image pickup element, is transmitted to the camera control unit. The camera control unit generates a video signal on the basis of the electric signal, and outputs the video signal to, for example, a liquid crystal display which is a display device, thus displaying an endoscopic image on the screen of the liquid crystal display.

The endoscope cleaning sheath 3 is formed as an elongated cylindrical member. The insertion section 2a of the endoscope 2 is detachably inserted into the endoscope cleaning sheath 3. Thereby, the endoscope cleaning sheath 3 is disposed in a manner to cover the entirety of the insertion section 2a of the endoscope 2.

The endoscope cleaning sheath 3 is mainly composed of a distal end cover 8 which is a cylindrical body, and a tube body 9 which is composed of a multi-lumen tube. The distal end cover 8 is fitted on a distal end portion of the tube body 9. The proximal end side of the tube body 9 is provided with an operation section coupling unit 81 which has a greater diameter than the tube body 9. One end of a gas supply tube 10 and one end of a liquid supply tube 11 are coupled to the operation section coupling unit 81. The distal end cover 8 and the tube body 9 may be integrally formed, or may be formed of the same material.

The other end of the gas supply tube 10 is connected to the gas feed pump (gas feed source) 4 via an opening/closing valve 12 and a pressure adjusting valve 13, which are provided at positions along the gas supply tube 10. The other end of the liquid supply tube 11 is connected to the liquid feed pump (liquid feed source) 5 via the opening/closing valve 12 and a liquid feed tank 14, which are provided at positions along the liquid supply tube 11.

The tube body 9, which is composed of the multi-lumen tube, is formed of a flexible material such as silicone, urethane or Teflon (trademark), or a rigid material such as polyamide, polyethylene, polypropylene or polycarbonate.

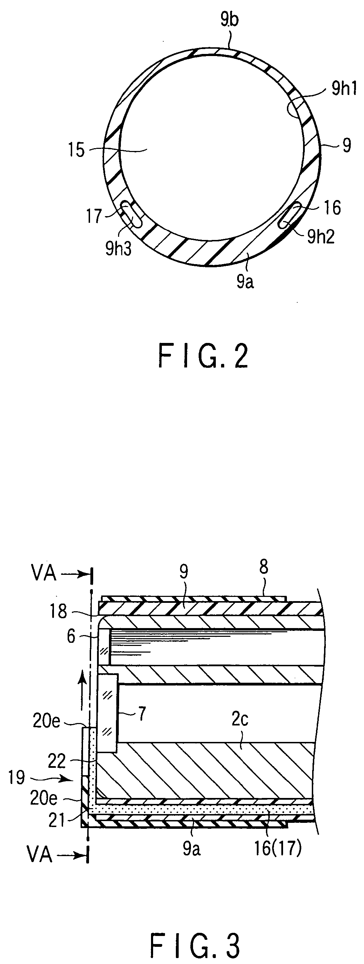

As shown in FIG. 2, the tube body 9 has an eccentric hole 9h1, the center axis of which is eccentric to the outer peripheral surface of the tube body 9. Thereby, the peripheral wall of the tube body 9, which defines the eccentric hole 9h1, includes a large thickness portion 9a and a small thickness portion 9b. The eccentric hole 9h1 of the tube body 9 is a through-hole having openings at a distal end face and a proximal end face of the tube body 9. The eccentric hole 9h1 is used as an endoscope hole 15 in which the insertion section 2a of the endoscope 2 is inserted.

The large thickness portion 9a of the tube body 9 includes two through-holes 9h2 and 9h3, which penetrate the large thickness portion 9a in the axial direction of the tube body 9. One through-hole 9h2 is used as a gas feed path 16 serving as a first flow path for supplying a gas such as air. The other through-hole 9h3 is used as a liquid feed path 17 serving as a second flow path for supplying a liquid such as water or a cleaning solution. The proximal end side of the gas feed path 16 communicates with the gas supply tube 10, and the proximal end side of the liquid feed path 17 communicates with the liquid supply tube 11.

As shown in FIG. 3, the distal end cover 8 of the endoscope cleaning sheath 3 is a circular cylindrical member. The distal end cover 8 is provided with an opening portion 18 at a part thereof that is opposed to the front surface of the distal end section body 2c of the endoscope 2.

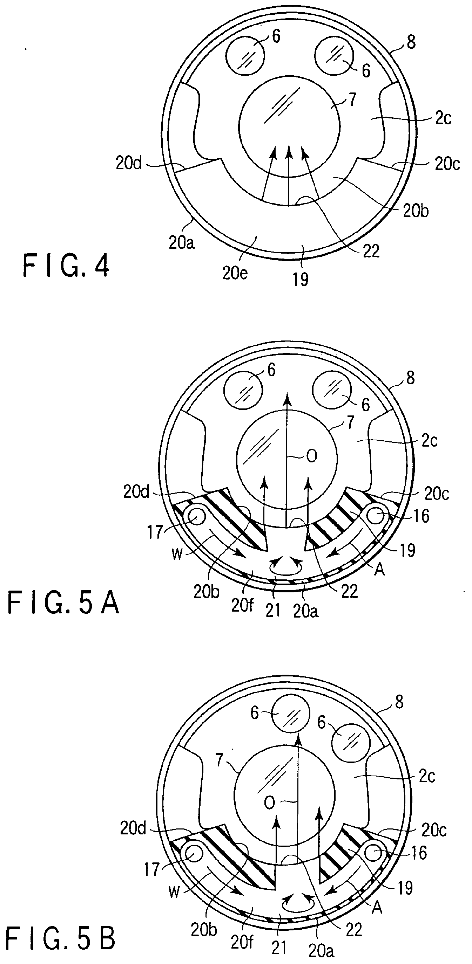

As shown in FIG. 3, a nozzle 19, which is bent inward in a substantially L shape, is integrally provided in a front end portion of the distal end cover 8. As shown in FIG. 4, the nozzle 19 has a space portion 20f which is surrounded by an outer peripheral wall 20a extending along the outer peripheral portion of the distal end section body 2c, an inner peripheral wall 20b surrounding a part of the outer periphery of the observation window 7, a right side wall 20c which defines a right side surface of the nozzle 19 in FIG. 4, a left side wall 20d which defines a left side surface of the nozzle 19, and an arcuate front wall 20e (see FIG. 3).

Further, the gas feed path 16 is open on the right wall 20c side in the space portion 20f of the nozzle 19. On the left wall 20d side, the liquid feed path 17 is open. Accordingly, as shown in FIG. 3, the gas feed path 16 and the liquid feed path 17 open toward the front end side of the distal end section body 2c of the endoscope 2, and are opposed to the inner surface of the front wall 20e of the nozzle 19.

A confluent portion 21 is provided in an intermediate part between the right side wall 20c and left side wall 20d of the nozzle 19. The flow of the gas (arrow A in FIG. 5A), which is supplied from the gas feed path 16 and flows in the space portion 20f of the nozzle 19, and the flow of the liquid (arrow W in FIG. 4), which is supplied from the liquid feed path 17 and flows in the space portion 20f of the nozzle 19, are made confluent in the confluent portion 21, and the gas and the liquid are caused to temporarily stay there and are mixed. Specifically, the gas and liquid flowing out of the gas feed path 16 and liquid feed path 17 collide with the inner surface of the front wall 20e, and are deflected and made confluent, thus reaching the confluent portion 21. Therefore, the gas and liquid are efficiently mixed.

The diameter of the flow path of the confluent portion 21 is set to be greater than the diameter of the flow path of each of the gas feed path 16 and liquid feed path 17. Thereby, the gas and the liquid collide with each other and are made turbulent in the confluent portion 21, and the gas and liquid are efficiently mixed.

Further, a jet outlet 22 is provided in the inner peripheral wall 20b of the nozzle 19, which is opposed to the confluent portion 21. The jet outlet 22 is formed of a rectangular elongated hole that is elongated in the longitudinal direction of the nozzle 19. Thereby, the gas/liquid mixture fluid, which is made confluent and mixed in the confluent portion 21, is jetted toward the observation window 7 and illumination windows 6 from the jet outlet 22.

The confluent portion 21 of the nozzle 19 having the above-described structure is provided on the plane that continuously extends to the observation window 7 of the distal end section body 2c. Thus, the gas (arrow A) that is supplied from the gas feed path 16 and the liquid (W) that is supplied from the liquid feed path 17 are mixed in the confluent portion 21, and an atomized gas/liquid mixture fluid is produced. This atomized gas/liquid mixture fluid is jetted toward the observation window 7 from the jet outlet 22. Thereby, contamination (mucus, blood, etc.) adhering to the observation window 7 is blown off and cleaned by the atomized gas/liquid mixture fluid that is jetted from the jet outlet 22. In the present embodiment, since the illumination windows 6 are also disposed on the continuous plane, contamination adhering to the illumination windows 6 can be blown off and cleaned at the same time by the atomized gas/liquid mixture fluid that is jetted from the jet outlet 22.

Next, the operation of the first embodiment is described. When the endoscope 1 is used, the endoscope cleaning sheath 3 is set in advance in the state in which the endoscope cleaning sheath 3 is fitted over the insertion section 2a of the endoscope 2. At this time, the entirety of the insertion section 2a is covered with the tube body 9. The distal end section body 2c of the endoscope 2 is covered with the distal end cover 8. The jet outlet 22 of the nozzle 19 of the distal end cover 8 is disposed to be directed to the observation window 7 and illumination windows 6 of the distal end section body 2c.

In the state in which the endoscope cleaning sheath 3 is fitted over the endoscope 2, the insertion section 2a of the endoscope 2, as one piece with the endoscope cleaning sheath 3, is inserted into a body cavity of a patient. The inside of the body cavity is observed by the endoscope 2, and a diseased part is treated, where necessary. At this time, there is a case in which contamination adheres to the observation window 7 and the field of vision is deteriorated. In this case, the observation window 7 can be cleaned remotely by the operation, which will be described below.

Specifically, at the time of the work of cleaning the observation window 7, the gas feed pump 4 is driven to feed gas and simultaneously the liquid feed pump 5 is driven to feed liquid. If the gas is fed from the gas feed pump 4, the gas is supplied to the gas feed path 16 via the gas supply tube 10. If the liquid is fed from the liquid feed pump 5, the liquid is supplied to the liquid feed path 17 via the liquid supply tube 11. Further, the gas in the gas feed path 16 and the liquid in the liquid feed path 17 are supplied into the space portion 20f of the nozzle 19. At this time, as shown in FIG. 5A, the flow A of the gas supplied from the gas feed path 16 and the flow W of the liquid supplied from the liquid feed path 17 collide and are made turbulent in the confluent portion 21, and the liquid and gas are mixed into an atomized gas/liquid mixture fluid. The atomized gas/liquid mixture fluid is jetted from the jet outlet 22 toward the observation window 7.

Since the confluent portion 21 of the nozzle 19 is provided on the plane that is continuous with the observation window 7 of the distal end section body 2c, the atomized gas/liquid mixture fluid, which is mixed in the confluent portion 21, can be jetted from the jet outlet 22 toward the observation window 7. As a result, the contamination adhering to the observation window 7 can efficiently be blown off and cleaned by the atomized gas/liquid mixture fluid that is jetted from the jet outlet 22 toward the observation window 7. Moreover, since the gas/liquid mixture fluid is the atomized fluid, particles of water are fine and immediately evaporate. Thus, no drops of water remain on the surface of the observation window 7. Therefore, the field of vision, as well as the illuminance, can instantaneously be secured.

In the case where the force of the atomized gas/liquid mixture fluid, which is jetted from the jet outlet 22, is to be increased, an adjustment knob 13a of the pressure adjusting valve 13 is controlled to increase the pressure of the gas. By this operation, the pressure of the gas that is supplied from the gas feed path 16 can be increased, and the gas/liquid mixture ratio can arbitrarily be controlled. In this case, in consideration of the difference in specific gravity between the gas and the liquid, the pressures of the gas feed pump 4 and liquid feed pump 5 may be preset to meet the relationship, i.e. the pressure of the gas feed pump 4 > the pressure of the liquid feed pump 5.

FIG. 5B shows a modification of the structure of FIG. 5A. In the first embodiment, as shown in FIG. 5A, the two illumination windows 6 are disposed to be symmetric in the right and left direction with respect to the center position of the observation window 7. Accordingly, the axis O of the mixture fluid, which is jetted from the jet outlet 22 of the nozzle 19, extends to the center of the observation window 7. On the other hand, in the present modification, as shown in FIG. 5B, the two illumination windows 6 are disposed at eccentric positions with eccentricity to the right side in FIG. 5B from the center position of the observation window 7. In the case of this positional relationship, the position of the nozzle 19 is varied, with the axis O of the mixture fluid that is jetted from the jet outlet 22 being displaced from the center of the observation window 7. Thereby, both the observation window 7 and illumination windows 6 can effectively be cleaned.

The above-described first embodiment is directed to the case in which the cleaning tube is fitted over the rigid endoscope. Needless to say, however, the invention is applicable to the case in which the cleaning tube is fitted over a flexible endoscope.

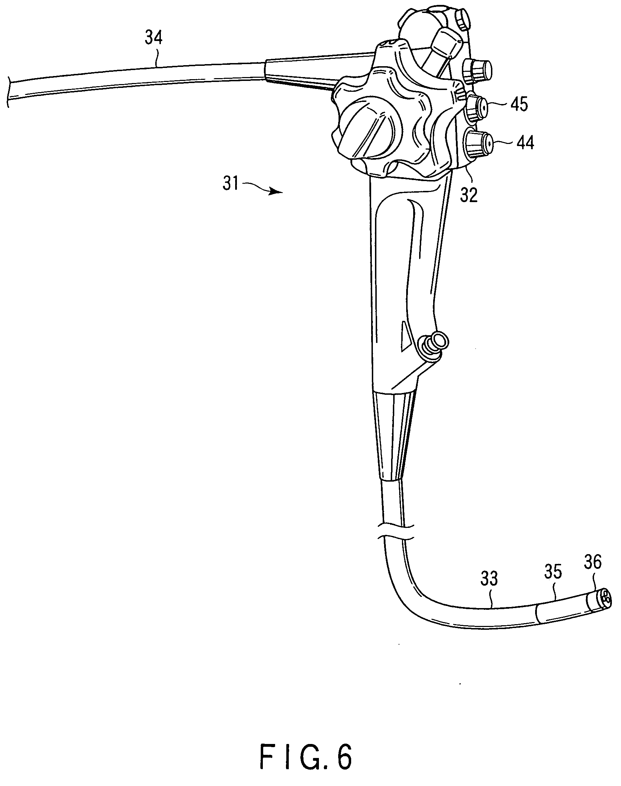

FIG. 6 to FIG. 8. show a second embodiment. In this embodiment, a flexible endoscope 31 shown in FIG. 6 is integrally equipped with a cleaning function. FIG. 6 is a perspective view showing the entirety of the flexible endoscope 31.

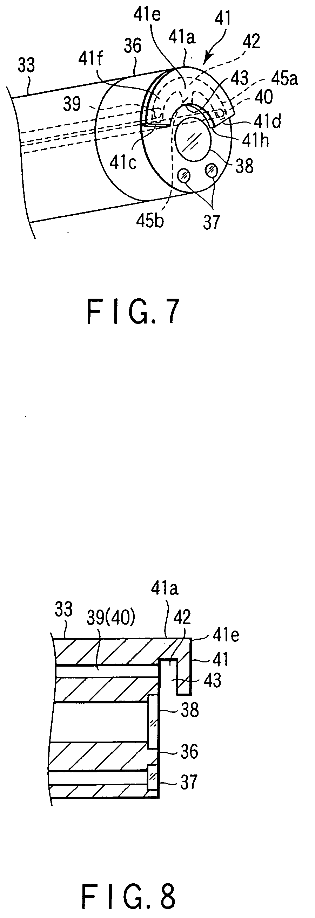

As shown in FIG. 6, in the flexible endoscope 31, a flexible insertion section 33 and a universal cord 34 are coupled to an operation section 32. A distal end section body 36 is provided on the insertion section 33 via a bending section 35. As shown in FIG. 7 and FIG. 8, the distal end section body 36 is provided with illumination windows 37 which constitute parts of an illumination optical system, and an observation window 38 which constitutes a part of an observation optical system. The illumination window 37 is connected to a light source device (not shown) via a light guide fiber. The observation optical system is provided with an image pickup device (not shown) including an image pickup element, such as a CCD, which photoelectrically converts an optical image, which is captured through the observation window 38, to an electric signal.

The insertion section 33 is provided with a gas feed path 39 for supplying a gas such as air, and a liquid feed path 40 for supplying a liquid such as water or a cleaning solution. Like the first embodiment, the gas feed path 39 and liquid feed path 40 communicate with the gas feed pump 4 and liquid feed pump 5 through the insertion section 33, operation section 32 and universal cord 34.

As shown in FIG. 7, an arcuate nozzle 41 is integrally provided at a front end portion of the distal end section body 36 of the endoscope 31 along the outer peripheral part of this front end portion.

An arcuate communication conduit 45a for communication between the gas feed path 39 and the liquid feed path 40 is formed in the nozzle 41. In the nozzle 41, an outer peripheral wall 41a of the communication conduit 45a is closed in a sealed state. A nozzle opening portion 45b is formed at a substantially central position in an inner peripheral wall 41b of the communication conduit 45a. Thereby, the communication conduit 45a of the nozzle 41 has a space portion 41f which is surrounded by the outer peripheral wall 41a extending along the outer peripheral portion of the distal end section body 36, the inner peripheral wall 41b surrounding a part of the outer periphery of the observation window 38, left and right end walls (left end wall 41c, right end wall 41d) in FIG. 7, and an arcuate front wall 41e. The space portion 41f is curved in an arcuate shape according to the curvature of the outer peripheral wall 41a and inner peripheral wall 41b. A taper portion, which is tapered toward the distal end opening, is formed in the nozzle opening portion 45b at the intermediate part of the space portion 41f. Thereby, a substantially T-shaped conduit is formed of the communication conduit 45a and nozzle opening portion 45b of the nozzle 41.

Further, the gas feed path 39 is open on the left end wall 41c side in the space portion 41f of the nozzle 41. On the right end wall 41d side, the liquid feed path 40 is open. Accordingly, the gas feed path 39 and the liquid feed path 40 are open to the front end side of the distal end section body 36, and are opposed to the inner surface of the front wall 41e of the nozzle 41.

An intermediate part in the longitudinal direction of the nozzle 41 is provided with a confluent portion 42 which combines and mixes the gas that is supplied from the gas feed path 39 and the liquid that is supplied from the liquid feed path 40. Further, the nozzle opening portion 45b of the inner peripheral wall 41b of the nozzle 41, which is opposed to the confluent portion 42, is provided with a jet outlet 43 of the nozzle 41. The jet outlet 43 is configured to jet the gas/liquid mixture fluid, which is made confluent and mixed in the confluent portion 21, toward the observation window 7.

The confluent portion 42 of the nozzle 41, which has the above-described structure, is provided on the same plane that is continuous with the observation window 38 provided on the distal end section body 36. The gas supplied from the gas feed path 39 and the liquid supplied from the liquid feed path 40 collide in the confluent portion 42. Thereby, the liquid and gas are made turbulent and mixed into an atomized gas/liquid mixture fluid is produced. The atomized gas/liquid mixture fluid is jetted from the jet outlet 43 toward the observation window 38. Thus, contamination adhering to the observation window 38 is blown off and cleaned by the atomized gas/liquid mixture fluid that is jetted from the jet outlet 43.

Further, the operation section 32 is provided with a gas/liquid feed button 44 and a suction button 45. The gas/liquid feed button 44 controls the flow amount of the gas supplied from the gas feed path 39 and the flow amount of the liquid supplied from liquid feed path 40, and controls the gas/liquid mixture fluid that is jetted in an atomized state from the jet outlet 43 of the nozzle 41. Like the first embodiment, the nozzle 41, observation window 38 and illumination windows 37 may successively be arranged on the same plane, so that the observation window 38 and illumination window 37 may be cleaned by the gas/liquid mixture fluid that is jetted from the nozzle 41 in the atomized state.

Next, the operation of the third embodiment is described. In the endoscope 31 of this embodiment, the nozzle 41 is integrally provided on the distal end section body 36 of the insertion section 33. The jet outlet 43 of the nozzle 41 is disposed on the same plane toward the observation window 38 and illumination windows 37. The insertion section 33 of the endoscope 31 is inserted into a body cavity of a patient, and the inside of the body cavity is observed and a diseased part is treated, where necessary. At this time, if contamination adheres to the observation window 38 and the field of vision is deteriorated, the observation window 38 can be cleaned remotely by the operation described below.

Specifically, the gas feed pump 4 is driven to feed gas and simultaneously the liquid feed pump 5 is driven to feed liquid. If the gas is fed from the gas feed pump 4, the gas is supplied to the gas feed path 39. Similarly, if the liquid is fed from the liquid feed pump 5, the liquid is supplied to the liquid feed path 40. Thus, the gas in the gas feed path 39 and the liquid in the liquid feed path 40 are supplied to the communication conduit 45a in the nozzle 41. The gas supplied from the gas feed path 39 and the liquid supplied from the liquid feed path 40 are mixed in the confluent portion 42, and an atomized gas/liquid mixture fluid is produced and jetted from the jet outlet 43 toward the observation window 38.

At this time, since the confluent portion 42 of the nozzle 41 is provided on the same plane that is continuous with the observation window 38 which is provided on the distal end section body 36, the atomized gas/liquid mixture fluid, which is mixed in the confluent portion 42, is jetted toward the observation window 38, and the contamination adhering to the observation window 38 can efficiently be blown off and cleaned. Moreover, since the jetted fluid is the atomized gas/liquid mixture fluid and particles of water immediately evaporate, no drops of water remain on the surface of the observation window 38, and the field of vision, as well as the illuminance, can instantaneously be secured.

In the case where the force of the atomized gas/liquid mixture fluid, which is jetted from the jet outlet 43, is to be increased, the gas/liquid feed button 44 that is provided on the operation section 32 is controlled to increase the pressure of the gas. Thereby, the pressure of the gas that is supplied from the gas feed path 39 can be increased, and the gas/liquid mixture ratio can arbitrarily be controlled.

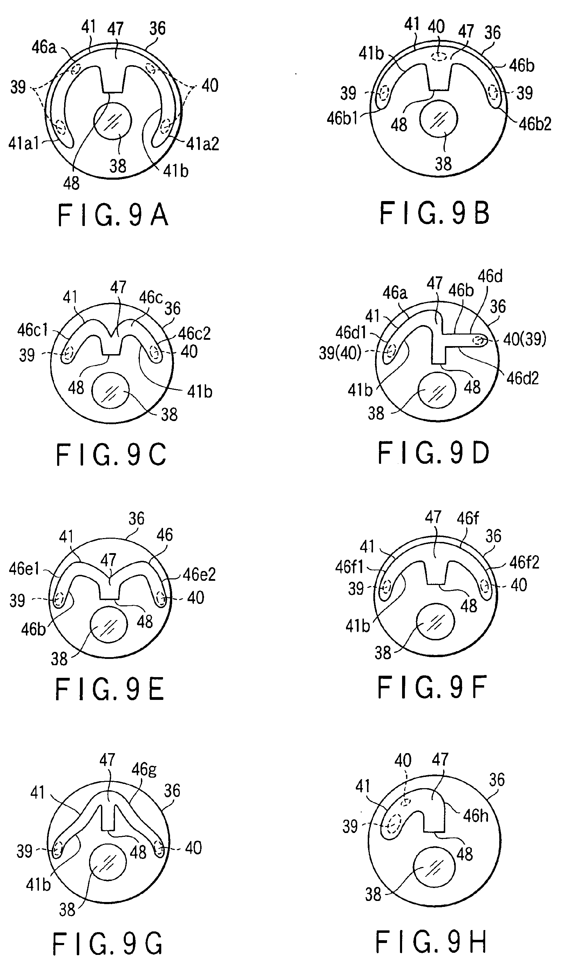

FIG. 9A to FIG. 9H show different modifications of the nozzle 41 in the second embodiment. Each of FIG. 9A to FIG. 9H is a schematic front view of the front end section body 36.

FIG. 9A shows a nozzle 41 according to a first modification of the second embodiment. The nozzle 41 shown in FIG. 9A is provided with a substantially C-shaped arcuate portion 46a having a curvature according to the outer peripheral part of the distal end section body 36. Extension portions 46a1 and 46a2 at both ends of the arcuate portion 46a are made to extend from the center line position of the front end portion of the distal end section body 36 of the endoscope 31 up to 90° or more to both sides, respectively.

In the inside of the nozzle 41, a confluent portion 47 is provided at an intermediate part of the arcuate portion 46a. Further, a jet outlet 48, which projects toward the observation window 38, is provided in an inner peripheral wall 41b of the nozzle 41 at the intermediate part of the arcuate portion 46a.

In the arcuate portion 46a, two gas feed paths 39 are open on the left side and two liquid feed paths 40 are open on the right side in such a manner that the two gas feed paths 39 and the two liquid feed paths 40 are symmetric with respect to the confluent portion 47 as a boundary. The gas supplied from the two gas feed paths 39 and the liquid supplied from the two liquid feed paths 40 collide in the confluent portion 47. Thereby, the gas and liquid are made turbulent and mixed into an atomized gas/liquid mixture fluid, and the atomized gas/liquid mixture fluid is jetted from the jet outlet 48 toward the observation window 38.

FIG. 9B shows a nozzle 41 according to a second modification of the second embodiment. The nozzle 41 shown in FIG. 9B is provided with an arcuate conduit 46b having a curvature according to the outer peripheral part of the distal end section body 36. The length of the conduit 46b is less than the length of the arcuate portion 46a of the first modification. Specifically, extension portions 46b1 and 46b2 at both ends of the conduit 46b are made to extend from the center line position of the front end portion of the distal end section body 36 of the endoscope 31 to 90° or less to both sides, respectively.

A confluent portion 47 is provided at an intermediate part of the conduit 46b. Further, a jet outlet 48, which projects toward the observation window 38, is provided in an inner peripheral wall 41b of the nozzle 41 at the intermediate part of the conduit 46b.

In the confluent portion 47, one liquid feed path 40 is open. Two gas feed paths 39 are open symmetrically in the extension portions 46b1 and 46b2 at both ends of the conduit 46b. The liquid supplied from the one liquid feed path 40 and the gas supplied from the two gas feed paths 39 collide in the confluent portion 47. Thereby, the gas and liquid are made turbulent and mixed into an atomized gas/liquid mixture fluid, and the atomized gas/liquid mixture fluid is jetted from the jet outlet 48 toward the observation window 38.

FIG. 9C shows a nozzle 41 according to a third modification of the second embodiment. The nozzle 41 shown in FIG. 9C is provided with a substantially M-shaped conduit 46c at the front end portion of the distal end section body 36 of the endoscope 31. This conduit 46c includes left and right conduits (left conduit 46c1 and right conduit 46c2), each of which is bent in a substantially V shape with an acute angle, on both sides of the center line position of the front end portion of the front end section body 36 of the endoscope 31.

A confluent portion 47 is provided at a coupling part between the left conduit 46c1 and right conduit 46c2. Further, a jet outlet 48, which projects toward the observation window 38, is provided in an inner peripheral wall 41b of the nozzle 41 at the coupling part between the left conduit 46c1 and right conduit 46c2. A gas feed path 39 is open in the left conduit 46c1, and a liquid feed path 40 is open in the right conduit 46c2. The liquid supplied from the liquid feed path 40 of the right conduit 46c2 and the gas supplied from the gas feed path 39 of the left conduit 46c1 collide in the confluent portion 47. Thereby, the gas and liquid are made turbulent and mixed into an atomized gas/liquid mixture fluid, and the atomized gas/liquid mixture fluid is jetted from the jet outlet 48 toward the observation window 38.

FIG. 9D shows a nozzle 41 according to a fourth modification of the second embodiment. The nozzle 41 shown in FIG. 9D includes a conduit 46d at a front end portion of the distal end section body 36 of the endoscope 31. The conduit 46d is composed of an inverted-V-shaped portion 46d1 and a horizontal portion 46d2 communicating with one end portion of the inverted-V-shaped portion 46d1.

The inverted-V-shaped portion 46d1 is formed in the state in which the inverted-V-shaped portion 46d1 is curved along the outer peripheral part of the distal end section body 36. A confluent portion 47 is provided at a bent part of the inverted-V-shaped portion 46d1. Further, a jet outlet 48, which projects toward the observation window 38, is provided in an inner peripheral wall 41b of the nozzle 41 at a coupling part between the inverted-V-shaped portion 46d1 and the horizontal portion 46d2. A gas feed path 39 (or a liquid feed path 40) is open on the left side of the conduit 46, and a liquid feed path 40 (or a gas feed path 39) is open on the right side. The liquid supplied from the liquid feed path 40 of the horizontal portion 46d2 and the gas supplied from the gas feed path 39 of the inverted-V-shaped portion 46d1 collide in the confluent portion 47. Thereby, the gas and liquid are made turbulent and mixed into an atomized gas/liquid mixture fluid, and the atomized gas/liquid mixture fluid is jetted from the jet outlet 48 toward the observation window 38.

FIG. 9E shows a nozzle 41 according to a fifth modification of the second embodiment. The nozzle 41 shown in FIG. 9E is provided with a substantially M-shaped conduit 46e at the front end portion of the distal end section body 36 of the endoscope 31. This conduit 46e includes left and right conduits (left conduit 46e1 and right conduit 46e2), each of which is bent in a substantially U shape with an obtuse angle, on both sides of the center line position of the front end portion of the front end section body 36 of the endoscope 31.

A confluent portion 47 is provided at a coupling part between the left conduit 46e1 and right conduit 46e2. Further, a jet outlet 48, which projects toward the observation window 38, is provided in an inner peripheral wall 41b of the nozzle 41 at the coupling part between the left conduit 46e1 and right conduit 46e2. A gas feed path 39 is open in the left conduit 46e1, and a liquid feed path 40 is open in the right conduit 46e2. The liquid supplied from the liquid feed path 40 of the right conduit 46e2 and the gas supplied from the gas feed path 39 of the left conduit 46e1 collide in the confluent portion 47. Thereby, the gas and liquid are made turbulent and mixed into an atomized gas/liquid mixture fluid, and the atomized gas/liquid mixture fluid is jetted from the jet outlet 48 toward the observation window 38.

FIG. 9F shows a nozzle 41 according to a sixth modification of the second embodiment. The nozzle 41 shown in FIG. 9F is provided with an arcuate conduit 46f having a curvature according to the outer peripheral part of the distal end section body 36. The length of the conduit 46f is less than the length of the arcuate portion 46a of the first modification. Specifically, extension portions 46f1 and 46f2 at both ends of the conduit 46f are made to extend from the center line position of the front end portion of the distal end section body 36 of the endoscope 31 to 90° or less to both sides, respectively.

A confluent portion 47 is provided at an intermediate part of the conduit 46f. Further, a jet outlet 48, which projects toward the observation window 38, is provided in an inner peripheral wall 41b of the nozzle 41 at the intermediate part of the conduit 46f.

A gas feed path 39 is open in the left-side extension portion 46f1 of the conduit 46f. A liquid feed path 40 is open in the right-side extension portion 46f2 of the conduit 46f. The liquid supplied from the liquid feed path 40 of the right-side extension portion 46f2 and the gas supplied from the gas feed path 39 of the left-side extension portion 46f1 collide in the confluent portion 47. Thereby, the gas and liquid are made turbulent and mixed into an atomized gas/liquid mixture fluid, and the atomized gas/liquid mixture fluid is jetted from the jet outlet 48 toward the observation window 38.

FIG. 9G shows a nozzle 41 according to a seventh modification of the second embodiment. The nozzle 41 shown in FIG. 9G is provided with a substantially angular conduit 46g. A confluent portion 47 is provided at an intermediate part of the conduit 46g. Further, a jet outlet 48, which projects toward the observation window 38, is provided in an inner peripheral wall 41b of the nozzle 41 at the intermediate part of the conduit 46g. A gas feed path 39 is open on the left side of the conduit 46g, and a liquid feed path 40 is open on the right side of the conduit 46g. The liquid supplied from the right-side liquid feed path 40 of the conduit 46g and the gas supplied from the left-side gas feed path 39 of the conduit 46g collide in the confluent portion 47. Thereby, the gas and liquid are made turbulent and mixed into an atomized gas/liquid mixture fluid, and the atomized gas/liquid mixture fluid is jetted from the jet outlet 48 toward the observation window 38.

FIG. 9H shows a nozzle 41 according to an eighth modification of the second embodiment. The nozzle 41 shown in FIG. 9H includes an inverted-V-shaped conduit 46h at a front end portion of the distal end section body 36 of the endoscope 31. A confluent portion 47 is provided at a bent part of the conduit 46h. Further, a jet outlet 48, which is open toward a side portion of the observation window 38 from the confluent portion 47, is provided. A large-diameter gas feed path 39 is open on a left-side end portion (on the upstream side) of the conduit 46h. A small-diameter liquid feed path 40 is open on the downstream side of the gas feed path 39. The liquid supplied from the left-side gas feed path 39 of the conduit 46h and the liquid supplied from the liquid feed path 40 collide in the confluent portion 47. Thereby, the gas and liquid are made turbulent and mixed into an atomized gas/liquid mixture fluid, and the atomized gas/liquid mixture fluid is jetted from the jet outlet 48 toward the observation window 38.

According to each of the modifications having the above-described structures, the following advantageous effects can be obtained. Specifically, the confluent portion 47 of the nozzle 41 is provided on the same plane as the observation window 38 that is provided on the distal end section body 36. Therefore, the atomized gas/liquid mixture fluid, which is mixed in the confluent portion 47, is jetted toward the observation window 38, and the contamination adhering to the observation window 38 can efficiently be blown off and cleaned. Moreover, since the jetted fluid is the atomized gas/liquid mixture fluid, no drops of water remain on the surface of the observation window 38, and the field of vision can instantaneously be secured.

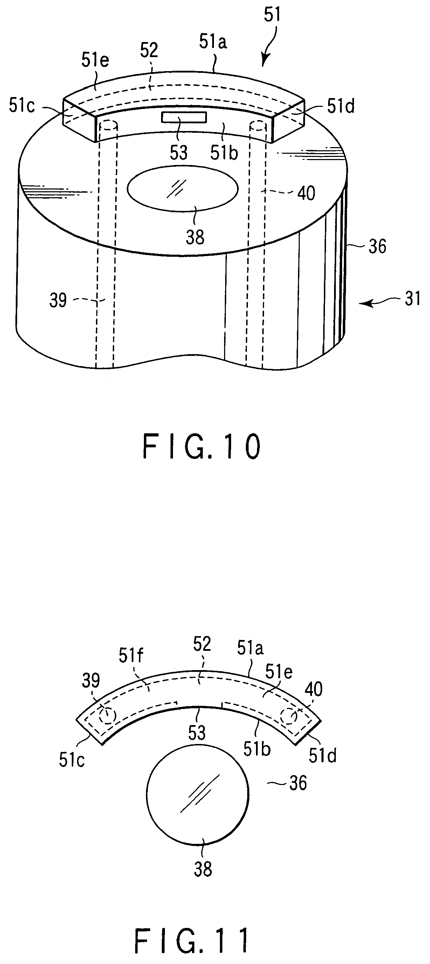

FIG. 10 and FIG. 11 show a third embodiment. The structural parts common to those in the second embodiment (see FIG. 6 to FIG. 8) are denoted by like reference numerals, and a description thereof is omitted.

An arcuate nozzle 51 is integrally provided at a front end portion of the distal end section body 36 of the endoscope 31 along the outer peripheral part of this front end portion. Specifically, the nozzle 51 includes a space portion (communication groove) 51f which is surrounded by an outer peripheral wall 51a extending along the outer peripheral portion of the distal end section body 36, left and right end walls (left end wall 51c, right end wall 51d) in FIG. 10, and an arcuate front wall 51e. The space portion 51f is curved in an arcuate shape according to the curvature of the outer peripheral wall 51a and inner peripheral wall 51b.

Further, the gas feed path 39 is open on the left end wall 51c side in the space portion 51f of the nozzle 51. On the opposite right end wall 51d side, the liquid feed path 40 is open. Accordingly, the gas feed path 39 and the liquid feed path 40 are open to the front end side of the distal end section body 36 of the endoscope 31, and are opposed to the inner surface of the front wall 51e of the nozzle 51.

An intermediate part in the longitudinal direction of the nozzle 51 is provided with a confluent portion 52 which combines and mixes the gas that is supplied from the gas feed path 39 and the liquid that is supplied from the liquid feed path 40. The flow path diameter of the confluent portion 52 is set to be greater than the flow path diameter of each of the gas feed path 39 and liquid feed path 40. Thereby, the gas and liquid collide and are made confluent in the confluent portion 52, and the gas and liquid are efficiently mixed. Further, a jet outlet 53, which is formed of a laterally elongated rectangular hole, is provided in the inner peripheral wall 51b of the nozzle 51, which is opposed to the confluent portion 52. The jet outlet 53 is configured to jet the gas/liquid mixture fluid, which is made confluent and mixed in the confluent portion 52, toward the observation window 38.

With the above-described structure, the following advantageous effects can be obtained. Specifically, the confluent portion 52 of the nozzle 51 is provided on the same plane as the observation window 38 that is provided on the distal end section body 36. The gas supplied from the air feed path 39 and the liquid supplied from the liquid feed path 40 are mixed in the confluent portion 52 into an atomized gas/liquid mixture fluid. The atomized gas/liquid mixture fluid is jetted from the jet outlet 53 toward the observation window 38. Thereby, the contamination adhering to the observation window 38 can be blown off and cleaned by the gas/liquid mixture fluid that is jetted from the jet outlet 53.

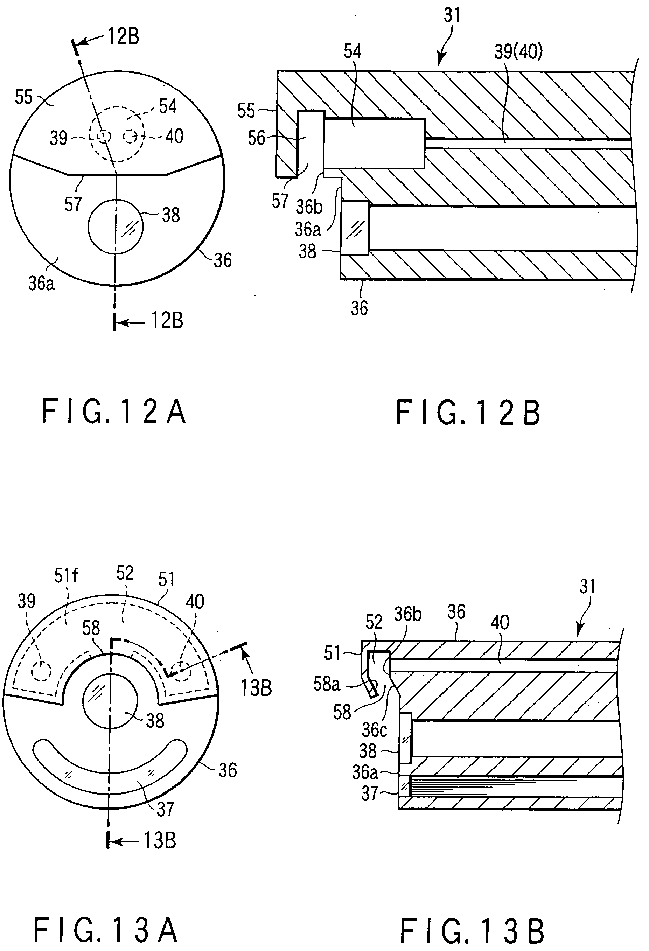

FIG. 12A and FIG. 12B show a fourth embodiment of the invention. The structural parts common to those in the second and third embodiments (see FIG. 6 to FIG. 8, FIG. 10 and FIG. 11) are denoted by like reference numerals, and a description thereof is omitted.

As shown in FIG. 12A, an observation window 38 is provided at a front end portion of the distal end section body 36 of the endoscope 31. As shown in FIG. 12B, a projection portion 36b, which projects forward from a plane 36a in which the observation window 38 is provided, is provided adjacent to the observation window 38. A nozzle 56 is formed in this projection portion 36b. The nozzle 56 is provided with a confluent portion 54, which is formed of a cylindrical recess portion that is formed in the distal end section body 36. A gas feed path 39 and a liquid feed path 40 are open at the bottom of the confluent portion 54.

The diameter of the confluent portion 54 is much greater than that of each of the gas feed path 39 and liquid feed path 40. Accordingly, when the liquid supplied from the gas feed path 39 and the liquid supplied from the liquid feed path 40 flow into the confluent portion 54, the liquid and gas are mixed in the confluent portion 54 with their flow speeds being decreased. At this time, since the gas from the gas feed path 39 and the liquid from the liquid feed path 40 collide with the inner surface of the front end wall 55 and are deflected and made confluent, the gas and liquid are efficiently mixed in the confluent portion 54.

The projection portion 36b of the distal end section body 36 is integrally provided with a front end wall portion 55 which is opposed to the opening part of the confluent portion 54. A jet outlet 57, which opens toward the observation window 38, is provided between the front wall portion 55 and the opening part of the confluent portion 54.

With the above-described structure, the following advantageous effects can be obtained. Specifically, the confluent portion 54 of the nozzle 56 and the jet outlet 57 are parallel to the observation window 38 that is provided on the distal end section body 36, and are provided in a manner to project forward from the observation window 38. The gas supplied from the air feed path 39 and the liquid supplied from the liquid feed path 40 are mixed in the confluent portion 54 into an atomized gas/liquid mixture fluid. The gas/liquid mixture fluid collides with the inner surface of the front end wall portion 55 of the nozzle 56 and is further mixed in an atomized state. The atomized gas/liquid mixture fluid is jetted from the jet outlet 57 toward the observation window 38. Thereby, the contamination adhering to the observation window 38 can efficiently be blown off and cleaned by the gas/liquid mixture fluid that is jetted from the jet outlet 57.

FIG. 13A and FIG. 13B show a fifth embodiment. The structural parts common to those in the second to fourth embodiments (see FIG. 6 to FIG. 12B) are denoted by like reference numerals, and a description thereof is omitted.

As shown in FIG. 13A, an observation window 38 and an illumination window 37 are provided at a front end portion of the distal end section body 36 of the endoscope 31. As shown in FIG. 13B, a projection portion 36b, which projects forward from a mount plane 36a in which the observation window 38 is provided, is provided adjacent to the observation window 38. An inclined surface 36c is formed between the mount plane 36a and the projection portion 36b.

The projection portion 36b is provided with a nozzle 51 which is integral with the distal end section body 36. The nozzle 51 has basically the same structure as in the third embodiment. Specifically, the nozzle 51 is curved in an arcuate shape according to the curvature of the outer peripheral surface of the distal end section body 36. Further, a confluent portion 52 is provided at an intermediate part of the nozzle 51. The nozzle 51 is provided with a jet outlet 58 which is parallel to the inclined surface 36c and has a fluid guide surface 58a which projects toward the observation window 38.

In addition, a space portion (conduit) 51f, which is curved in an arcuate shape, is formed in the nozzle 51. The confluent portion 52 and jet outlet 58 are disposed at the center position of the space portion 51f. Besides, a gas feed path 39 and a liquid feed path 40 are open at both ends of the space portion 51f. The gas feed path 39 and liquid feed path 40 are open toward the front end side of the distal end section body 36 of the endoscope 31, and are opposed to the inner surface of the nozzle 51. Accordingly, the gas from the gas feed path 39 and the liquid from the liquid feed path 40 collide with the inner surface of the nozzle 51 and are deflected and made confluent, thus reaching the confluent portion 52. Therefore, the gas and liquid are efficiently mixed.

With the above-described structure, the following advantageous effects can be obtained. Specifically, the confluent portion 52 of the nozzle 51 and the jet outlet 58 are parallel to the observation window 38 that is provided on the distal end section body 36, and are provided in a manner to project forward from the observation window 38. The gas supplied from the air feed path 39 and the liquid supplied from the liquid feed path 40 are mixed in the confluent portion 52 into a gas/liquid mixture fluid. The gas/liquid mixture fluid collides with the inner surface of the nozzle 51 and is further mixed in an atomized state. The atomized gas/liquid mixture fluid is jetted from the jet outlet 58 toward the observation window 38. Thereby, the contamination adhering to the observation window 38 can be blown off and cleaned by the gas/liquid mixture fluid that is jetted from the jet outlet 58.

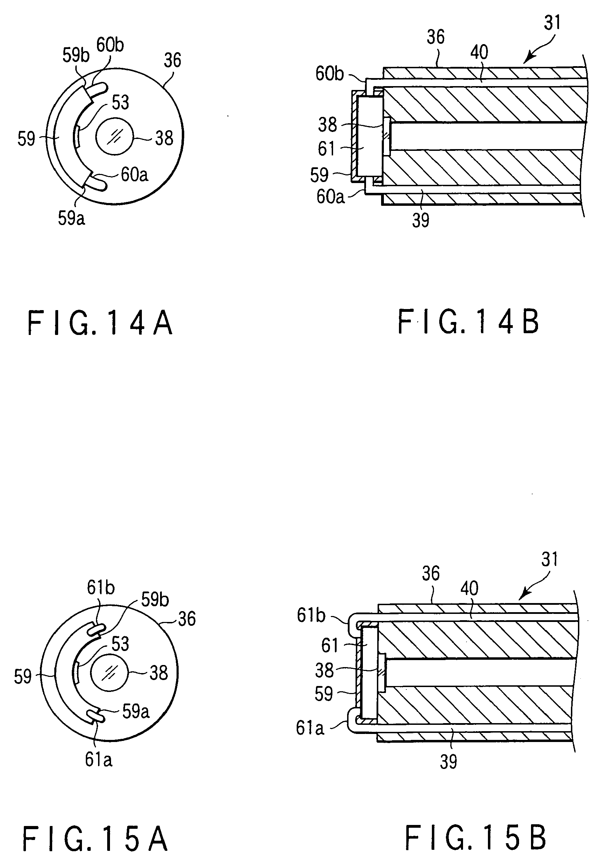

FIG. 14A and FIG. 14B show a sixth embodiment. The structural parts common to those in the second to fifth embodiments (see FIG. 6 to FIG. 13B) are denoted by like reference numerals, and a description thereof is omitted.

A front end portion of the distal end section body 36 of the endoscope 31 includes an arcuate nozzle 59 which has the same structure as the nozzle 51 of the third embodiment (see FIG. 10 and FIG. 11) and is curved in an arcuate shape along the outer peripheral part of the distal end section body 36. The nozzle 59 includes a space portion which is surrounded by walls. As shown in FIG. 14B, an L-shaped conduit 60a, which is connected to the gas feed path 39, penetrates one end wall 59a (on the lower side in FIG. 14A) of the nozzle 59 and is open to the inside of the nozzle 59. Thereby, the nozzle 59 communicates with the L-shaped conduit 60a. An L-shaped conduit 60b, which is connected to the liquid feed path 40, penetrates the opposite-side end wall 59b (on the upper side in FIG. 14A) of the nozzle 59 and is open to the inside of the nozzle 59. Thereby, the nozzle 59 communicates with the L-shaped conduit 60b. Accordingly, the opening portions of the gas feed path 39 and the liquid feed path 40 are opposed to a confluent portion 61 at an intermediate part of the nozzle 59.

Further, a jet outlet 53, which is directed to the observation window 38, is provided at a central position of the inner peripheral wall of the nozzle 59. The jet outlet 53 is disposed at a position facing the confluent portion 61. Thus, the jet outlet 53 is configured to jet the gas/liquid mixture fluid, which is made confluent and mixed in the confluent portion 61, toward the observation window 38.

With the above-described structure, the following advantageous effects can be obtained. Specifically, the confluent portion 61 of the nozzle 59 is provided on the same plane as the observation window 38 that is provided on the distal end section body 36. The gas supplied from the air feed path 39 and the liquid supplied from the liquid feed path 40 are mixed in the confluent portion 61 into an atomized gas/liquid mixture fluid. The atomized gas/liquid mixture fluid is jetted from the jet outlet 53 toward the observation window 38. Thereby, the contamination adhering to the observation window 38 can be blown off and cleaned by the gas/liquid mixture fluid that is jetted from the jet outlet 53.

FIG. 15A and FIG. 15B show a seventh embodiment. The structural parts common to those in the second and sixth embodiments (see FIG. 6 to FIG. 14B) are denoted by like reference numerals, and a description thereof is omitted.

A front end portion of the distal end section body 36 of the endoscope 31 includes an arcuate nozzle 59 which has the same structure as the nozzle 59 of the sixth embodiment (see FIG. 14A and FIG. 14B) and is curved in an arcuate shape along the outer peripheral part of the distal end section body 36. The nozzle 59 includes a space portion which is surrounded by walls. A U-shaped conduit 61a, which is connected to the gas feed path 39, penetrates a front surface portion of the nozzle 59 on one end wall 59a side (on the lower side in FIG. 15A) of the nozzle 59 and is open to the inside of the nozzle 59. Thereby, the nozzle 59 communicates with the U-shaped conduit 61a. A U-shaped conduit 61b, which is connected to the liquid feed path 40, penetrates a front surface portion of the nozzle 59 on the opposite end wall 59b side (on the upper side in FIG. 15A) of the nozzle 59 and is open to the inside of the nozzle 59. Thereby, the nozzle 59 communicates with the U-shaped conduit 61b. Accordingly, the opening portions of the gas feed path 39 and the liquid feed path 40 are opposed to the distal end face of the distal end section body 36. Thus, the fluids from the gas feed path 39 and liquid feed path 40 once collide with the distal end surface of the distal end section body 36 and are then mixed.

Further, a confluent portion 61 is provided at an intermediate part of the nozzle 59. A jet outlet 53 is provided at a central position of the inner peripheral wall of the nozzle 59. The jet outlet 53 is disposed at a position facing the confluent portion 61. Thus, the jet outlet 53 is configured to jet the gas/liquid mixture fluid, which is made confluent and mixed in the confluent portion 61, toward the observation window 38.

With the above-described structure, the following advantageous effects can be obtained. Specifically, the confluent portion 61 of the nozzle 59 is provided on the same plane as the observation window 38 that is provided on the distal end section body 36. The gas supplied from the air feed path 39 and the liquid supplied from the liquid feed path 40 are mixed in the confluent portion 61 into an atomized gas/liquid mixture fluid. The atomized gas/liquid mixture fluid is jetted from the jet outlet 53 toward the observation window 38. As a result, the contamination adhering to the observation window 38 can be blown off and cleaned by the gas/liquid mixture fluid that is jetted from the jet outlet 53.

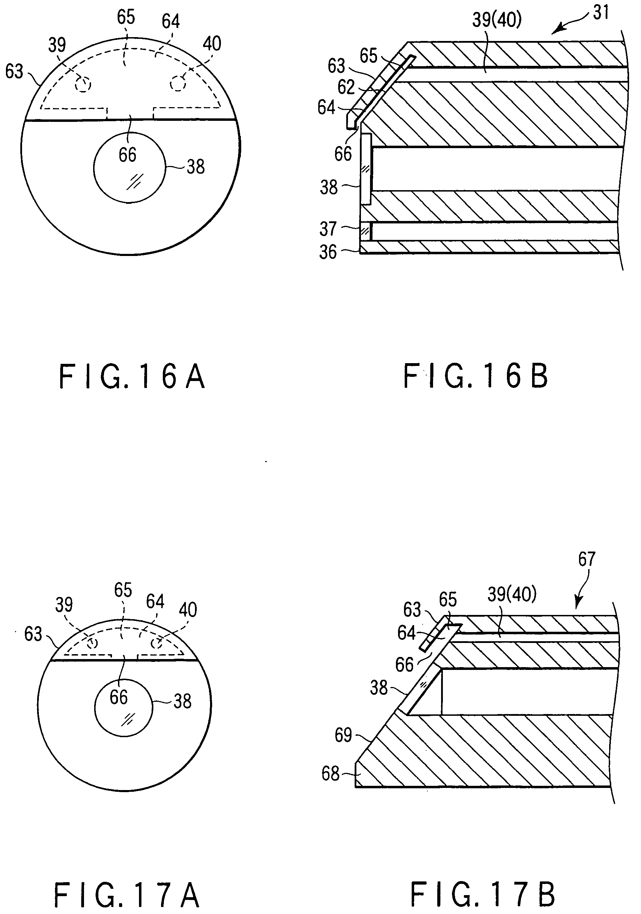

FIG. 16A and FIG. 16B show an eighth embodiment of the invention. The structural parts common to those in the second to seventh embodiments (see FIG. 6 to FIG. 15B) are denoted by like reference numerals, and a description thereof is omitted.

As shown in FIG. 16B, an inclined surface 62 is provided on one side portion of the distal end section body 36 of the endoscope 31. An inclined nozzle 63 is provided to be integral with the distal end section body 36 along the inclined surface 62. A space portion 64 is provided in the inclined nozzle 63 along the inclined surface 62. In FIG. 16A, the gas feed path 39 is open to the left end part of the space portion 64, and the liquid feed path 40 is open to the right end part of the space portion 64. Thus, the opening portions of the gas feed path 39 and liquid feed path 40 are opposed to the inner surface of the inclined nozzle 63.

Further, as shown in FIG. 16A, a confluent portion 65 is provided at a central position of the space portion 64 of the inclined nozzle 63. A jet outlet 66 is provided to be opposed to the confluent portion 65. Thus, the jet outlet 66 is configured to jet the gas/liquid mixture fluid, which is made confluent and mixed in the confluent portion 65, toward the observation window 38.

With the above-described structure, the following advantageous effects can be obtained. Specifically, the confluent portion 65 of the inclined nozzle 63 is provided on the rear side of the observation window 38 that is provided on the distal end section body 36. The gas supplied from the air feed path 39 and the liquid supplied from the liquid feed path 40 are mixed in the confluent portion 65 into an atomized gas/liquid mixture fluid. The atomized gas/liquid mixture fluid is jetted from the jet outlet 66 toward the observation window 38. Thereby, the contamination adhering to the observation window 38 can be blown off and cleaned by the gas/liquid mixture fluid that is jetted from the jet outlet 66.

FIG. 17A and FIG. 17B show a ninth embodiment of the invention. The structural parts common to those in the second to eighth embodiments (see FIG. 6 to FIG. 16B) are denoted by like reference numerals, and a description thereof is omitted.

The present embodiment is applied to an oblique-viewing endoscope 67. As shown in FIG. 17B, an inclined surface 69 is provided on a front end portion of a distal end section body 68 of the oblique-viewing endoscope 67. An observation window 38 is provided on the inclined surface 69. On one side portion of the inclined surface 69, an inclined nozzle 63 is provided to be integral with the distal end section body 68 along the inclined surface 69. The inclined nozzle 63 has basically the same structure as in the eighth embodiment (see FIG. 16A and FIG. 16B). A space portion 64 is provided along the inclined surface 69. The gas feed path 39 and liquid feed path 40 are open to the space portion 64. Thus, the opening portions of the gas feed path 39 and liquid feed path 40 are opposed to the inner surface of the inclined nozzle 63. Further, a confluent portion 65 is provided in the space portion 64 of the inclined nozzle 63. A jet outlet 66 is provided to be opposed to the confluent portion 65. Thus, the jet outlet 66 is configured to jet the gas/liquid mixture fluid, which is made confluent and mixed in the confluent portion 65, toward the observation window 82.

With the above-described structure, the following advantageous effects can be obtained. Specifically, the confluent portion 65 of the inclined nozzle 63 is provided on the same plane as the observation window 82 that is provided on the distal end section body 68. Thereby, the gas supplied from the air feed path 39 and the liquid supplied from the liquid feed path 40 are mixed in the confluent portion 65 into an atomized gas/liquid mixture fluid. The atomized gas/liquid mixture fluid is jetted from the jet outlet 66 toward the observation window 82. Thereby, the contamination adhering to the observation window 82 can be blown off and cleaned by the gas/liquid mixture fluid that is jetted from the jet outlet 66.

In the second to ninth embodiments, the flexible endoscopes have been described. Needless to say, the invention is also applicable to rigid endoscopes.

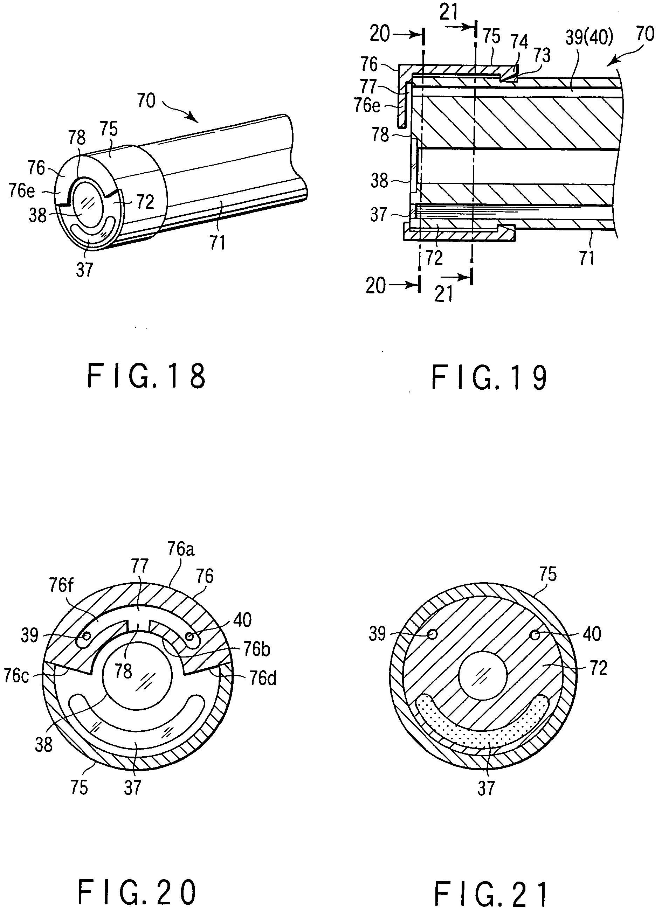

FIG. 18 to FIG. 21 show a tenth embodiment. The structural parts common to those in the second to ninth embodiments (see FIG. 6 to FIG. 17B) are denoted by like reference numerals, and a description thereof is omitted.

The present embodiment shows a distal end cap-equipped endoscope. An observation window 38 and an illumination window 37 are provided on a distal end section body 72 of an insertion section 71 of a flexible endoscope 70. In addition, as shown in FIG. 19 and FIG. 21, a gas feed path 39 and a liquid feed path 40 are provided in the insertion section 71. Openings of the gas feed path 39 and liquid feed path 40 are provided on a front end face of the distal end section body 72.

Further, an annular engagement groove 73 is provided on an outer peripheral surface of the distal end section body 72. A distal end cap 75 is detachably attached to the distal end section body 72. The distal end cap 75 is a circular cylindrical member. An engagement projection 74, which is detachably engaged with the engagement groove 73, is provided on an inner peripheral surface of the rear end portion of the distal end cap 75.

In the front end portion of the distal end cap 75, an arcuate nozzle 76 is integrally provided along the outer peripheral portion of the distal end cap 75. Specifically, as shown in FIG. 20, the nozzle 76 includes a space portion 76f which is surrounded by an outer peripheral wall 76a extending along the outer peripheral portion of the distal end cap 75, an inner peripheral wall 76b surrounding a part of the outer periphery of the observation window 38, left and right end walls (left end wall 76c, right end wall 76d) in FIG. 20, and an arcuate front wall 76e. The space portion 76f is curved in an arcuate shape according to the curvature of the outer peripheral wall 76a and inner peripheral wall 76b.

Further, the gas feed path 39 is open on the left end wall 76c side in the space portion 76f of the nozzle 76. On the opposite right end wall 76d side, the liquid feed path 40 is open. Accordingly, the gas feed path 39 and the liquid feed path 40 are open to the front end side of the distal end cap 75, and are opposed to the inner surface of the front wall 76e of the nozzle 76.

An intermediate part in the longitudinal direction of the space portion 76f of the nozzle 76 is provided with a confluent portion 77 which combines and mixes the gas that is supplied from the gas feed path 39 and the liquid that is supplied from the liquid feed path 40. Further, a jet outlet 78 is provided in the inner peripheral wall 76b of the nozzle 76, which is opposed to the confluent portion 77. The jet outlet 78 is configured to jet the gas/liquid mixture fluid, which is made confluent and mixed in the confluent portion 77, toward the observation window 38. Specifically, the gas from the gas feed path 39 and the liquid from the liquid feed path 40 collide with the inner surface of the front wall 76e and are deflected and made confluent. Since the gas from the gas feed path 39 and the liquid from the liquid feed path 40 reach the confluent portion 77 in this state, the gas and liquid are efficiently mixed in the confluent portion 77.

With the above-described structure, the following advantageous effects can be obtained. Specifically, the confluent portion 77 of the nozzle 76 is provided on the same plane as the observation window 38 that is provided on the distal end section body 72. Thereby, the gas supplied from the air feed path 39 and the liquid supplied from the liquid feed path 40 are mixed in the confluent portion 77 into an atomized gas/liquid mixture fluid. The atomized gas/liquid mixture fluid is jetted from the jet outlet 78 toward the observation window 38. Thereby, the contamination adhering to the observation window 38 can be blown off and cleaned by the gas/liquid mixture fluid that is jetted from the jet outlet 78.

The present invention is not limited directly to the above-described embodiments. In practice, the structural elements can be modified and embodied without departing from the invention. Various inventions can be made by properly combining the structural elements disclosed in the embodiments. For example, some structural elements may be omitted from all the structural elements disclosed in the embodiments. Furthermore, structural elements in different embodiments may properly be combined.

## Claims

1. A distal end section (36) of an insertion section (33) configured to be inserted in a body cavity, comprising:
a distal end section body (36) which constitutes the distal end section (36) and has at least an observation window (38);
a liquid feed path (40) configured to supply a liquid to the distal end section body (36) side and communicate with a liquid feed source (5);
a gas feed path (39) configured to supply a gas to the distal end section body (36) side and communicate with a gas feed source (4); and
an inclined nozzle (63) which is provided in the distal end section body (36) and configured to clean the observation window (38) by jetting toward the observation window (38) a mixture fluid in which the liquid supplied from the liquid feed path (40) and the gas supplied from the gas feed path (39) are mixed, wherein the inclined nozzle (63) includes
a confluent portion (65) configured to mix and make confluent the liquid supplied from the liquid feed path (40) and the gas supplied from the gas feed path (39); and
a jet outlet (66) configured to jet a gas/liquid mixture fluid generated in the confluent portion (65) towards the observation window (38),
**characterized in that** an inclined surface (62) is provided on a side portion of the distal end section body (36), the inclined nozzle (63) being integral with the distal end section body (36) along the inclined surface (62),
a space portion (64) is provided in the inclined nozzle (63) along the inclined surface (62),
opening portions of the gas feed path (39) and the liquid feed path (40) are provided opposed to an inner surface of the inclined nozzle (63), and
the confluent portion (65) is provided at a central position of the space portion (64) within the inclined nozzle (63) opposed to the jet outlet (66) and on the rear side of the observation window (38).

2. The distal end section (36) of claim 1, **characterized in that** the confluent portion (65) is a space which has a greater fluid path diameter than each of the liquid feed path (40) and the gas feed path (39), and is configured to mix the liquid supplied from the liquid feed path (40) and the gas supplied from the gas feed path (39) and cause the liquid and the gas to temporarily stay therein.

3. The distal end section (36) of any of the preceding claims, **characterized by** being one of a distal end section (36) of an insertion section (33) of an endoscope, a distal end section (9) of an insertion section (9) of an endoscope cleaning sheath (3), and a distal end cap (75) detachably attached to the distal end section (72) of the insertion section of the endoscope (31).

## Patentansprüche

1. Distaler Endabschnitt (36) eines Einführabschnitts (33), der dazu eingerichtet ist, in einen Körperhohlraum eingeführt zu werden, mit:
einem distalen Endabschnittskörper (36), der den distalen Endabschnitt (36) bildet und mindestens ein Beobachtungsfenster (38) hat;
einem Flüssigkeitszufuhrpfad (40) der dazu eingerichtet ist, einer Seite des distalen Endabschnittskörpers (36) eine Flüssigkeit zuzuführen und mit einer Flüssigkeitszufuhrquelle (5) zu kommunizieren;
einem Gaszufuhrpfad (39), der dazu eingerichtet ist, einer Seite des distalen Endabschnittskörpers (36) ein Gas zuzuführen und mit einer Gaszufuhrquelle (4) zu kommunizieren; und
einer geneigten Düse (63), die in dem distalen Endabschnittskörper (36) vorgesehen und dazu eingerichtet ist, das Beobachtungsfenster (38) zu reinigen, indem sie ein Mischfluid in Richtung des Beobachtungsfensters (38) spritzt, in dem die von dem Flüssigkeitszufuhrpfad (40) zugeführte Flüssigkeit und das von dem Gaszufuhrpfad (39) zugeführte Gas gemischt sind, wobei die geneigte Düse umfasst einen Zusammenführungsabschnitt (65), der dazu eingerichtet ist, die von dem Flüssigkeitszufuhrpfad (40) zugeführte Flüssigkeit und das von dem Gaszufuhrpfad (39) zugeführte Gas zu mischen und zusammenzuführen; und
einen Spritzauslass (66), der dazu eingerichtet ist, das in dem Zusammenführungsabschnitt (65) erzeugte Gas/Flüssigkeits-Mischfluid in Richtung des Beobachtungsfensters (38) zu spritzen,
**dadurch gekennzeichnet, dass**
eine geneigte Oberfläche (62) an einem Seitenabschnitt des distalen Endabschnittskörpers (36) vorgesehen ist, wobei die geneigte Düse (63) entlang der geneigten Oberfläche (62) integriert mit dem distalen Endabschnittskörper (36) ausgebildet ist,
ein Raumabschnitt (64) in der geneigten Düse (63) entlang der geneigten Oberfläche (62) vorgesehen ist,
Öffnungsabschnitte des Gaszufuhrpfads (39) und des Flüssigkeitszufuhrpfads (40) gegenüber einer Innenfläche der geneigten Düse (63) vorgesehen sind, und
der Zusammenführungsabschnitt (65) an einer zentralen Position des Raumabschnitts (64) innerhalb der geneigten Düse (63) gegenüber dem Spritzauslass (66) und auf der Rückseite des Beobachtungsfensters (38) vorgesehen ist.

2. Distaler Endabschnitt (36) nach Anspruch 1, **dadurch gekennzeichnet, dass** der Zusammenflussabschnitt (65) ein Raum ist, der einen größeren Fluidpfaddurchmesser hat als sowohl der Flüssigkeitszufuhrpfad (40) als auch der Gaszufuhrpfad (39) und dazu eingerichtet ist, die von dem Flüssigkeitszufuhrpfad (40) zugeführte Flüssigkeit und das von dem Gaszufuhrpfad (39) zugeführte Gas zu mischen und die Flüssigkeit und das Gas dazu zu veranlassen, temporär darin zu verweilen.

3. Distaler Endabschnitt nach einem der vorhergehenden Ansprüche, **dadurch gekennzeichnet, dass** er ein distaler Endabschnitt (36) eines Einführabschnitts (33) eines Endoskops, ein distaler Endabschnitt (9) eines Einführabschnitts (9) einer Endoskopreinigungshülse (3) oder eine distale Endkappe (75) ist, die lösbar an dem distalen Endabschnitt (72) des Einführabschnitts des Endoskops (31) befestigt ist.

## Revendications

1. Section d'extrémité distale (36) d'une section d'insertion (33) configurée pour être insérée dans une cavité de corps, comprenant :
un corps de section d'extrémité distale (36) qui constitue la section d'extrémité distale (36) et comporte au moins une fenêtre d'observation (38) ;
un trajet d'alimentation en liquide (40) configuré pour délivrer un liquide vers le côté du corps de section d'extrémité distale (36) et communiquer avec une source d'alimentation en liquide (5) ;
un trajet d'alimentation en gaz (39) configuré pour délivrer un gaz vers le côté du corps de section d'extrémité distale (36) et communiquer avec une source d'alimentation en gaz (4) ; et
une buse inclinée (63) qui est prévue dans le corps de section d'extrémité distale (36) et configurée pour nettoyer la fenêtre d'observation (38) en faisant gicler vers la fenêtre d'observation (38) un fluide de mélange dans lequel le liquide délivré depuis le trajet d'alimentation en liquide (40) et le gaz délivré depuis le trajet d'alimentation en gaz (39) sont mélangés, dans lequel la buse inclinée (63) comprend une partie confluente (65) configurée pour mélanger et faire confluer le liquide délivré depuis le trajet d'alimentation en liquide (40) et le gaz délivré depuis le trajet d'alimentation en gaz (39) ; et
un orifice de sortie en jet (66) configuré pour faire gicler un fluide de mélange gaz/liquide généré dans la partie confluente (65) vers la fenêtre d'observation (38),
**caractérisée en ce que**
une surface inclinée (62) est prévue sur une partie latérale du corps de section d'extrémité distale (36), la buse inclinée (63) étant solidaire avec le corps de section d'extrémité distale (36) le long de la surface inclinée (62),
une partie d'espace (64) est prévue dans la buse inclinée (63) le long de la surface inclinée (62),
des parties d'ouverture du trajet d'alimentation en gaz (39) et du trajet d'alimentation en liquide (40) sont prévues opposées à une surface interne de la buse inclinée (63), et
la partie confluente (65) est prévue au niveau d'une position centrale de la partie d'espace (64) à l'intérieur de la buse inclinée (63) opposée à l'orifice de sortie en jet (66) et sur le côté arrière de la fenêtre d'observation (38).

2. Section d'extrémité distale (36) selon la revendication 1, **caractérisée en ce que** la partie confluente (65) est un espace qui présente un diamètre de trajet fluidique plus grand que chacun parmi le trajet d'alimentation en liquide (40) et le trajet d'alimentation en gaz (39), et est configurée pour mélanger le liquide délivré depuis le trajet d'alimentation en liquide (40) et le gaz délivré depuis le trajet d'alimentation en gaz (39) et amener le liquide et le gaz à stagner temporairement.

3. Section d'extrémité distale (36) selon l'une quelconque des revendications précédentes, **caractérisée en ce qu'**elle est l'une parmi une section d'extrémité distale (36) d'une section d'insertion (33) d'un endoscope, une section d'extrémité distale (9) d'une section d'insertion (9) d'une gaine de nettoyage d'endoscope (3), et un capuchon d'extrémité distale (75) attaché de manière détachable à la section d'extrémité distale (72) de la section d'insertion de l'endoscope (31).
